# FASCICULE DE BREVET EUROPEEN

(11) **EP 1 633 700 B1**
(45) Date de publication et mention de la délivrance du brevet: **25.04.2012**
(21) Numéro de dépôt: 04742667.1
(22) Date de dépôt: 07.05.2004
(51) Int. Cl.: C07C 255/04, C07C 253/34, C07C 253/10

(54) **PROCEDE DE PREPARATION DE DINITRILES**
VERFAHREN ZUR HERSTELLUNG VON DINITRIL
DINITRILE PREPARATION METHOD

(30) Priorité: 12.05.2003 FR 0305672
(43) Date de publication de la demande: 15.03.2006
(73) Titulaire: Invista Technologies S.à.r.l., 9000 St. Gallen (CH)
(72) Inventeur: ROSIER, Cécile, F-69510 Soucieu en Jarrest (FR); LECONTE, Philippe, F-69330 Meyzieu (FR); BOURGEOIS, Damien, F-69003 Lyon (FR)
(74) Mandataire: Cockerton, Bruce Roger
(86) Numéro de dépôt international: PCT/FR2004/001108
(87) Numéro de publication internationale: WO 2004/101497

(56) Documents cités:
- WO-A-03/011457
- US-A- 4 539 302

## Description

La présente invention concerne un procédé de préparation de dinitriles par hydrocyanation de composés nitriles insaturés en présence d'un catalyseur à base d'élément métallique à l'état d'oxydation zéro et de ligands organophosphorés.

Elle se rapporte plus particulièrement à un procédé de récupération d'un catalyseur d'hydrocyanation de nitriles insaturés en dinitriles à partir du milieu d'hydrocyanation.

La réaction d'hydrocyanation est industriellement utilisée pour la synthèse de composés comprenant des fonctions nitriles à partir de composés comprenant des insaturations. Ainsi, l'adiponitrile qui est un intermédiaire chimique important notamment dans la fabrication de l'hexaméthylène diamide, monomère pour un certain nombre de polymères comme le polyamide, est fabriqué par hydrocyanation en deux étapes du butadiène ou d'une coupe d'hydrocarbures appelée coupe C4 comprenant du butadiène. Dans ce procédé de fabrication, les deux réactions sont mises en oeuvre avec des systèmes catalytiques essentiellement composés par les mêmes espèces, à savoir un complexe organométallique de coordination et au moins un ligand organophosphoré de type organophosphite monodentate tel que le tritolylphosphite.

De nombreux brevets décrivent ce procédé de fabrication de l'adiponitrile, ainsi que les procédés de fabrication des catalyseurs.
Par ailleurs, pour l'économie du procédé, il est important de pouvoir récupérer le système catalytique et le recycler dans les étapes d'hydrocyanation.
Ainsi, le brevet US 4539302 décrit un procédé de récupération du catalyseur à partir du milieu réactionnel obtenu dans la deuxième étape du procédé de préparation de l'adiponitrile, à savoir l'hydrocyanation de nitriles insaturés en dinitriles.

Ce procédé de récupération par décantation permet de limiter les pertes en élément métallique et facilite le contrôle du rapport ligand organophosphoré/élément métallique faible pour l'hydrocyanation de nitriles insaturés. Ainsi, on peut, également, récupérer un système catalytique avec un rapport ligand/élément métallique élevé permettant un recyclage et une réutilisation du système catalytique dans les étapes de fabrication de catalyseur et/ou dans les étapes d'hydrocyanation du butadiène ou l'isomérisation des pentènenitriles ramifiés.

De nombreux autres ligands organophosphorés ont été proposés pour la catalyse de ces réactions d'hydrocyanation.

Ainsi, les ligands bidentates de type organophosphite, organophosphinite, organophosphonite et organophosphine ont été décrits dans de nombreux brevets tels que, par exemple, les brevets WO9906355, WO9906356, WO9906357, WO9906358, WO9952632, WO9965506, WO9962855, US5693843, WO961182, WO9622968, US5981772, WO0136429, WO9964155, W00213964.

Enfin, il a également été proposé par le brevet WO 03/11457 d'utiliser un mélange de ligands mono et bidentate pour la catalyse des réactions d'hydrocyanation. L'utilisation d'un tel mélange permet de faciliter la synthèse du catalyseur et la formation du complexe organométallique, notamment par rapport aux procédés de synthèse des complexes organométalliques avec des ligands bidentates.

Dans le cas de mélanges de ligands, il est également important de pouvoir récupérer le catalyseur sans perdre de ligands ni d'élément métallique.

Un des buts de la présente invention est de proposer dans un procédé de préparation de dinitriles, un procédé de récupération d'un catalyseur formé par un mélange de ligands mono et bidentate et de pouvoir réutiliser le catalyseur ainsi récupéré dans les étapes d'hydrocyanation et/ou d'isomérisation.

A cet effet, l'invention propose un procédé de préparation de composés dinitriles par hydrocyanation de composés mononitriles insaturés en présence d'un système catalytique comprenant un complexe organométallique formé à partir d'un élément métallique et d'un mélange de ligands organophosphorés comprenant au moins un composé organophosphite monodentate et au moins un composé organophosphoré bidentate choisi dans le groupe comprenant les organophosphites, les organophosphinites, les organophosphonites, les organophosphines, et éventuellement un promoteur, ledit procédé comprenant une récupération du système catalytique, caractérisé en ce qu'il consiste à contrôler la concentration en nitriles insaturés dans le milieu réactionnel issu de la réaction d'hydrocyanation pour obtenir une concentration pondérale en nitriles insaturés inférieure à 20 % dans le dit milieu, à alimenter le dit milieu dans une étape de décantation en deux phases supérieure et inférieure, à séparer les deux phases, la phase inférieure étant au moins en partie, recyclée à ladite étape d'hydrocyanation, la phase supérieure étant soumise à une extraction liquide/liquide pour séparer le complexe organométallique et les composés organophosphorés présents dans ladite phase supérieure des dinitriles.

Comme ligand monodentate organophosphite convenable pour l'invention, on peut citer, à titre d'exemple, le triphénylphosphite, le tritolylphosphite (TTP), le tricyménylphosphite.

Comme ligands bidentates convenables pour l'invention, on peut citer les composés de structures suivantes dans lesquelles Ph signifie phényle :

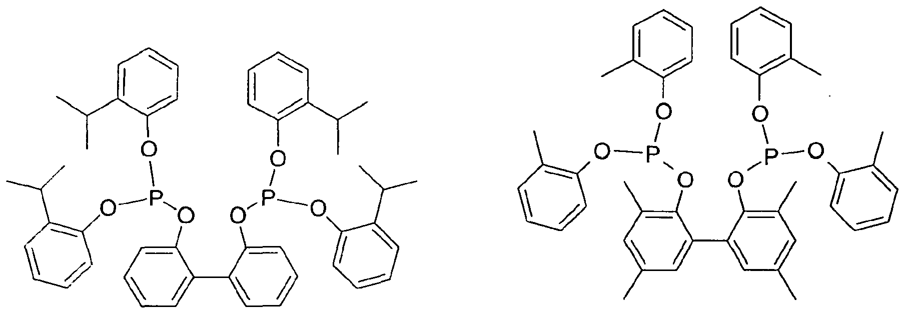

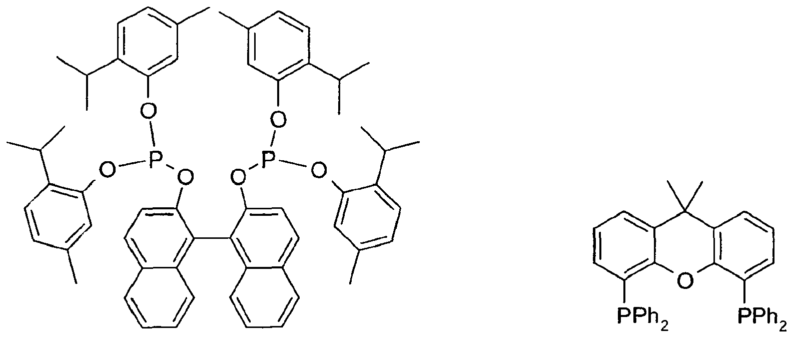

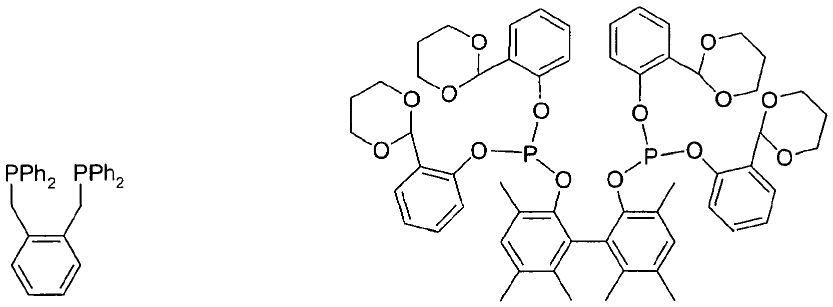

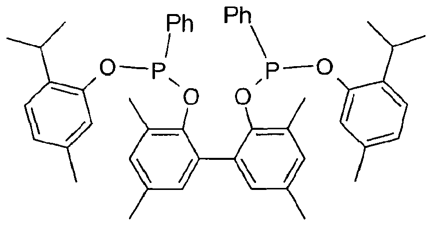

Selon une caractéristique préférentielle de l'invention, le catalyseur présent dans le milieu réactionnel comprend généralement un nombre de moles de ligand bidentate exprimé en nombre d'atome de phosphore par rapport à un atome d'élément métallique compris entre 1 et 4, tandis de celui de ligand monodentate exprimé en nombre d'atome de phosphore est compris entre 4 et 7.
Dans le présent texte l'expression ligand/nickel se rapporte toujours au rapport de la totalité des molécules de ligands mono et/ou bidentates par rapport au nombre d'atome de nickel, sauf indication explicite différente.

Les éléments métalliques présentant un effet catalytique dans une réaction hydrocyanation sont, par exemple, le nickel, le cobalt, le fer, le ruthénium, le rhodium, le palladium, l'osmium, l'iridium, le platine, le cuivre, l'argent, l'or, le zinc, le cadmium, le cérium. Le nickel est l'élément catalytique préféré. Pour plus de clarté, l'élément métallique sera désigné par le terme nickel dans la suite du présent texte, sans que cela est une signification limitative.

Par ailleurs, dans la réaction d'hydrocyanation des nitriles insaturés, un promoteur ou cocatalyseur est généralement utilisé. Comme promoteurs préférés, les acides de Lewis sont généralement utilisés.

A titre d'exemple, On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont avantageusement choisis parmi les composés des éléments des groupes Ib, IIb, IIIa, IIIb, IVa, IVb, Va, Vb, Vlb, Vllb et VIII de la Classification périodique des éléments. Ces composés sont le plus souvent des sels, notamment des halogénures, comme chlorures ou bromures, sulfates, sulfonates, halogenoalkylsulfonates, perhalogénoalkylsulfonates, notamment fluoroalkylsulfonates ou perfluoroalkylsulfonates, halogénoacétates, perhalogénoacétates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thullium, l'ytterbium et le lutétium, le chlorure de cobalt, le chlorure ferreux, le chlorure d'yttrium.

On peut également utiliser comme acide de Lewis des composés comme le triphénylborane, le tétraisopropylate de titane.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux, le triphénylborane, le trifluorométhylsulfonate d'indium, le trifluoroacétate d'indium, et les mélanges chlorure de zinc/chlorure stanneux.
Le cocatalyseur acide de Lewis mis en oeuvre représente généralement de 0,005 à 50 moles par mole de nickel.

Selon une caractéristique préférée de l'invention, le milieu alimenté à l'étape de décantation est refroidi à une température comprise entre 25°C et 75 °C, avantageusement entre 30°C et 55°C

Pour obtenir une décantation et séparation en deux phases du milieu réactionnel, il est avantageux que la concentration pondérale en nickel dans le milieu réactionnel alimenté à l'étape de décantation soit comprise entre 0,2 et 2 %.

Pour obtenir une telle concentration en nickel, il peut être nécessaire d'ajouter une certaine quantité de système catalytique dans le milieu réactionnel sortant de l'étape d'hydrocyanation. En effet, la concentration en nickel utilisé dans le milieu réactionnel quand il est associé à un ligand bidentate peut être très faible comme, par exemple, de l'ordre de 100 à 2000 mg de nickel/kg de milieu réactionnel.

Selon un mode de réalisation préféré de l'invention, la concentration en nickel dans le milieu issu de l'étape d'hydrocyanation est ajustée à une valeur convenable et désirée par introduction d'au moins une partie de la phase inférieure obtenue à l'étape de décantation.

Selon encore une autre caractéristique préférée de l'invention, la concentration en nitriles insaturés dans le milieu alimenté à l'étape de décantation est inférieure ou égale à 20% en poids, avantageusement comprise entre 4 et 20% en poids.

Le contrôle ou l'obtention d'une telle concentration peut être réalisé selon différentes manières :
- Une première possibilité est de contrôler et définir le taux de transformation des nitriles insaturés à l'étape d'hydrocyanation pour obtenir dans le milieu réactionnel en fin de réaction ou sortant du dit réacteur, une concentration en nitriles insaturés inférieure ou égale à 20 % en poids.
- Selon un autre mode de réalisation de l'invention, le milieu réactionnel sortant de l'étape d'hydrocyanation est alimenté dans une étape de distillation sous pression réduite ou flashage des nitriles insaturés non transformés. Cette étape permet de contrôler la concentration des nitriles insaturés dans le milieu alimenté à l'étape de décantation.

Pour éviter des pertes en nickel par précipitation dans le pied de distillation, il est avantageux que la température de pied de cette étape de distillation soit inférieure à 140°C. Par température de pied de distillation on entend la température du milieu se trouvant dans le bouilleur mais également la température des parois dudit bouilleur.

Dans ce mode de réalisation comprenant l'étape de distillation d'une partie des nitriles insaturés, le réglage ou contrôle de la concentration en nickel du pied de distillation alimenté à l'étape de décantation peut être réalisé par recyclage d'une partie de la phase inférieure de la décantation. Ce recyclage est avantageusement réalisé avant l'alimentation du milieu dans l'étape de distillation.

Selon le procédé de l'invention, la phase inférieure obtenue dans le décanteur ou phase lourde contient la majeure partie du nickel et du ligand bidentate ainsi qu'une partie du ligand monodentate. Toutefois, comme les coefficients de partage de l'élément métallique et des ligands organophosphorés entre les deux phases sont différents, le rapport molaire de ligands/atomes d'élément métallique sera faible dans la phase inférieure et élevé dans la phase supérieure. En outre, le procédé de l'invention permet d'utiliser la plus grande partie du ligand bidentate préférentiellement dans l'étape d'hydrocyanation des nitriles insaturés pour ainsi obtenir une sélectivité élevée en dinitriles linéaires.
Selon une autre caractéristique du procédé de l'invention, la phase supérieure récupérée à l'étape de décantation contient un rapport molaire ligands/nickel exprimé en nombre d'atome de phosphore avantageusement supérieur à 8.

Selon l'invention, la récupération totale du catalyseur et des ligands est effectuée par une extraction liquide/liquide de ceux-ci au moyen d'un solvant d'extraction non miscible avec les dinitriles.

Comme solvant d'extraction convenable, on peut citer à titre d'exemple, les hydrocarbures aliphatiques saturés linéaires ou cycliques comme l'hexane, l'heptane, octane, le cyclohexane, le cyclopentane, le cycloheptane et plus généralement les cycloparaffines ou analogues. Le cyclohexane est le solvant d'extraction préféré.

Après extraction, la solution cyclohexanique du complexe organométallique et des ligands est alimentée dans une étape d'évaporation ou de distillation du solvant d'extraction. Pour limiter la perte de nickel par précipitation, il est important que le rapport molaire ligand/nickel exprimé en nombre d'atome de phosphore par rapport au nombre d'atome de nickel soit élevé, et notamment supérieur à 8. Ce rapport élevé est, dans le procédé de l'invention, obtenu dans l'étape de décantation grâce aux coefficients de partage des différentes espèces chimiques entre les deux phases supérieure et inférieure.

En outre, pour limiter les pertes de nickel, la température de pied de l'étape de distillation ou évaporation sous pression atmosphérique ou sous une pression supérieure à la pression atmosphérique est avantageusement inférieure à 180°C. Par température de pied, on entend la température du milieu contenu dans le bouilleur de la dite étape ainsi que la température des parois dudit bouilleur.

Le procédé de récupération du catalyseur de l'invention permet donc une récupération totale du catalyseur et des ligands organophosphorés.

L'invention s'applique généralement à un procédé de transformation de mononitriles insaturés en dintrile constituant la seconde étape d'un procédé de fabrication de composés dinitriles par double hydrocyanation de dioléfines telles que le butadiène. Ce procédé comprend une première étape d'hydrocyanation de la dioléfine en nitriles insaturés mise en oeuvre avec un catalyseur qui est constitué, avantageusement, par les mêmes composés que ceux du système catalytique utilisé dans la seconde étape avec éventuellement des rapports entre les composés différents. A cette première étape est généralement associé une réaction d'isomérisation des mononitriles ramifiés formés pour les transformer en mononitriles insaturés linéaires qui seront alimentés en seconde étape. Cette isomérisation est mise en oeuvre avec un système catalytique équivalent à celui de la première étape et en absence de cyanure d'hydrogène.

Dans un tel procédé comprenant deux étapes, le catalyseur et les ligands récupérés après évaporation du solvant d'extraction sont avantageusement recyclés soit dans une étape d'hydrocyanation des dioléfines (ou d'une coupe d'hydrocarbures C4), soit dans une étape de préparation du catalyseur et/ou à l'isomérisation des nitriles insaturés ramifiés ou encore ajoutés au catalyseur récupéré à partir de la phase inférieure de décantation avant introduction dans l'étape d'hydrocyanation des composés nitriles insaturés.

Dans un mode de réalisation de l'invention, au moins une partie du catalyseur récupéré après évaporation du solvant d'extraction est introduit dans l'étape d'isomérisation des nitriles ramifiés puis éventuellement dans l'étape d'hydrocyanation des diènes. Le catalyseur récupéré après cette dernière étape peut être recyclé directement dans l'étape d'extraction liquide/liquide ou l'étape de décantation.
Dans ce mode de réalisation, le catalyseur utilisé dans les étapes d'isomérisation et d'hydrocyanation des diènes comprend une faible proportion de ligand bidentate permettant ainsi de diminuer les pertes en ce ligand dues par exemple à une réaction d'hydrolyse ou avec des composés contenus dans les diènes tels que le tertiobutyl catéchol (TBC) présent dans le butadiène.

La présente invention s'applique de manière préférentielle à l'hydrocyanation de nitriles insaturés, linéaires ou ramifiés, comprenant de 3 à 8 atomes de carbone et plus préférentiellement de 3-pentènenitrile et/ou
4-pentènenitrile pour la production d'adiponitrile avec utilisation d'un catalyseur du type représenté par la formule suivante :

### Ni (L₁)ₓ (L₂)_{y}

Dans laquelle L₁ représente un ligand monodentate et L₂ un ligand bidentate.
x et y représentent des nombres décimaux allant de 0 à 4, la somme x +2y étant égale à 3 ou 4. Le catalyseur peut être constitué d'un mélange de complexes répondant à la formule générale ci-dessus.
Le système catalytique ou le milieu réactionnel peut également comprendre une quantité de ligand organophosphorés mono et/ou bidentate sous forme libre, c'est-à-dire non lié au nickel

Les systèmes catalytiques de l'invention peuvent être obtenus par formation dans une première étape d'un complexe organométallique entre le nickel et le ligand monodentate. Des procédés de formation de tels complexes sont par exemple, décrits dans les brevets US 3903120 et US 4,416,825.
Dans une seconde étape, le ligand bidentate est ajouté dans le milieu contenant le dit complexe organométallique.

Les première et seconde étapes d'hydrocyanation sont avantageusement réalisées en série.
Dans ce cas, il est avantageux qu'au moins une partie du catalyseur récupéré, notamment celui récupéré à l'extraction liquide/liquide, et plus particulièrement le catalyseur récupéré dans la phase supérieure de l'étape de décantation, soit recyclée et utilisée comme catalyseur dans la première étape d'hydrocyanation du butadiène, et/ou à l'étape d'isomérisation des nitriles insaturés ramifiés en nitriles insaturés linéaires. L'utilisation d'un système catalytique identique ou similaire pour l'hydrocyanation du butadiène et celle des pentènenitriles est préférée.

Toutefois, il est également possible d'utiliser le système catalytique décrit ci-dessus uniquement dans l'étape d'hydrocyanation des nitriles insaturés, le système catalytique utilisé dans la première étape d'hydrocyanation du butadiène et l'étape d'isomérisation étant différent par la nature des composés.

D'autres détails, avantages de l'invention apparaîtront plus clairement au vu des exemples donnés ci-dessous uniquement à titre indicatif et d'illustration.

### Abréviations :

PN : pentènenitriles
DN : dinitriles (mélanges de dinitriles AdN, MGN, ESN contenant majoritairement de l'AdN)
AdN : adiponitrile
MGN : méthyl glutaronitrile
ESN : éthylsuccinonitrile

### Exemple 1 :

Une hydrocyanation de pentènenitriles a été effectuée en utilisant un système catalytique contenant un complexe organométallique obtenu à partir de nickel à l'état d'oxydation zéro, d'un promoteur ZnCl₂ et de composés organophosphorés de formule suivantes :

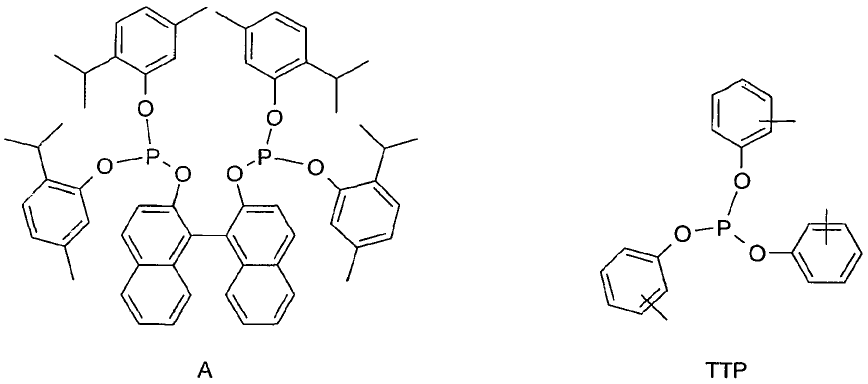

Après flashage des PN, le milieu réactionnel obtenu a la composition suivante (% exprimé en poids, les rapports sont des rapports molaires):
Ni = 0.58%
TTP/Ni = 5
Ligand A/Ni = 1,2
P/Ni = 7,4
DN = 64,0%
PN = 6,4%
ZnCl₂ = 0.16%
Le mélange est refroidi jusqu'à 50°C : On observe une séparation en deux phases liquides. La phase dense contient environ 83% du Ni(0) et environ 73% du ligand **A**. Le rapport TTP/Ni est environ de 3 dans la phase inférieure et environ de 15 dans la phase supérieure. Le rapport molaire TTP/ligand **A** est proche de 8 dans la phase supérieure.
La phase supérieure qui contient de l'adiponitrile, est soumise à une extraction liquide/liquide avec du cyclohexane. La solution cyclohexanique obtenue est évaporée. La récupération du système catalytique comme culot de l'évaporation est quantitative.

### Exemple 2 : Décantation du système mixte TTP/ligand B

L'exemple 1 est répété mais en remplaçant le ligand **A** par le ligand **B** de formule suivante :

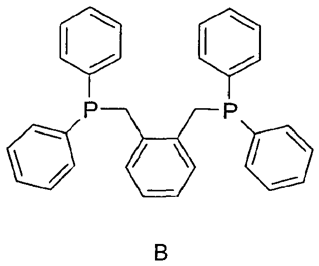

Après flashage des PN, le milieu réactionnel obtenu a la composition suivante (% exprimé en poids, les rapports sont des rapports molaires):
Ni = 0.70%
TTP/Ni = 4
Ligand B/Ni = 1
P/Ni = 6
DN = 67.7%
PN = 6.7%
ZnCl₂ = 0.17%

, Le milieu est refroidi jusqu'à 50°C: on observe la séparation en deux phases liquides. La phase dense contient environ 70% du Ni(0) et environ 70% du ligand **B**. Le rapport TTP/Ni est inférieur à 2 dans la phase dense et environ égal à 9 dans la phase légère. Le rapport TTP/ligand **B** est proche de 9 dans la phase légère.
La phase supérieure est soumise à une extraction liquide/liquide avec du cyclohexane. La solution cyclohexanique est évaporée. La récupération du système catalytique comme culot de l'évaporation est quantitative.

## Revendications

1. Procédé de préparation de composés dinitriles par hydrocyanation de composés mononitriles insaturés en présence d'un système catalytique comprenant un complexe organométallique formé à partir d'un élément métallique et d'un mélange de ligands organophosphorés comprenant au moins un composé organophosphite monodentate et au moins un composé organophosphoré bidentate choisi dans le groupe comprenant les organophosphites, les organophosphinites, les organophosphonites, les organophosphines, et éventuellement un promoteur, ledit procédé comprenant une récupération du système catalytique, **caractérisé en ce qu'**il consiste à contrôler la concentration en nitriles insaturés, dans le milieu réactionnel issu de la réaction d'hydrocyanation pour obtenir une concentration pondérale en nitriles insaturés inférieure à 20 % dans le dit milieu, à alimenter le dit milieu dans une étape de décantation en deux phases supérieure et inférieure, à séparer les deux phases, la phase inférieure étant, au moins en partie, recyclée à une étape d'hydrocyanation, la phase supérieure étant soumise à une extraction liquide/liquide pour extraire le complexe organométallique et les composés organophosphorés présents dans ladite phase.

2. Procédé selon la revendication 1, **caractérisé en ce que** le milieu réactionnel est refroidi à une température comprise entre 25°C et 75°C avant d'être introduit dans l'étape de décantation.

3. Procédé selon l'une des revendications 1 ou 2, **caractérisé en ce que** la concentration pondérale en nickel dans le milieu alimenté à l'étape de décantation est comprise entre 0,2 et 2%

4. Procédé selon l'une des revendications précédentes, **caractérisé en ce que** la concentration pondérale en nitriles insaturés dans le milieu alimenté à l'étape de décantation est comprise entre 4 % et 20 % en poids.

5. Procédé selon l'une des revendications précédentes, **caractérisé en ce que** la concentration en nitriles insaturés dans le milieu réactionnel issu de l'étape d'hydrocyanation est inférieure à 20 % en poids.

6. procédé selon l'une de revendication 1 à 5, **caractérisé en ce que** le milieu réactionnel issu de l'étape d'hydrocyanation est alimenté dans une étape de distillation des nitriles insaturés, le pied de distillation étant alimenté dans l'étape de décantation.

7. Procédé selon l'une des revendications 1 à 6, **caractérisé en ce que** la phase inférieure de l'étape de décantation est, au moins partiellement, recyclée dans le milieu réactionnel issu de l'hydrocyanation.

8. Procédé selon la revendication 7, **caractérisé en ce qu'**une partie de ladite phase inférieure est recyclée dans le milieu réactionnel issu de l'hydrocyanation, avant l'étape de distillation des nitriles insaturés.

9. procédé selon l'une des revendications précédentes, **caractérisé en ce que** le nombre de moles de ligand bidentate exprimé en nombre d'atome de phosphore par rapport à un atome d'élément métallique est compris entre 1 et 4 dans le milieu d'hydrocyanation.

10. Procédé selon l'une des revendications précédentes, **caractérisé en ce que** le nombre de moles de ligand monodentate exprimé en nombre d'atome de phosphore par rapport à un atome d'élément métallique dans le milieu d'hydrocyanation. est compris entre 4 et 7.

11. Procédé selon l'une des revendications précédentes, **caractérisé en ce que** le solvant utilisé pour mettre en oeuvre l'extraction liquide/liquide du catalyseur et des composés organophosphorés contenus dans la phase supérieure est choisi dans le groupe comprenant les hydrocarbures aliphatiques ou cycloaliphatiques, saturés ou non.

12. Procédé selon l'une des revendications précédentes, **caractérisé en ce que** le catalyseur et les ligands organophosphorés extraits dans l'étape d'extraction liquide/liquide sont récupérés par évaporation du solvant d'extraction.

13. Procédé selon la revendication 11 et 12, **caractérisé en ce que** le solvant d'extraction est choisi dans le groupe comprenant l'hexane, l'heptane, octane, le cyclohexane, le cyclopentane, le cycloheptane.

14. Procédé selon l'une des revendications précédentes, **caractérisé en ce que** le nombre de moles de ligands organophosphorés exprimé en nombre d'atome de phosphore dans le milieu alimenté à l'étape d'extraction liquide/liquide par rapport à un atome d'élément métallique est supérieur à 8.

15. Procédé selon l'une des revendications précédentes, **caractérisé en ce que** le ligand monodentate est un composé choisi dans le groupe comprenant le triphénylphosphite, le tritolylphosphite, le tricyménylphosphite.

16. Procédé selon l'une des revendications précédentes, **caractérisé en ce que** le ligand organophosphoré bidentate est choisi dans le groupe comprenant les organophosphites organophosphonites, organophosphinites, organophosphines.

17. Procédé selon la revendication 16, **caractérisé en ce que** le ligand organophosphoré bidentate est choisi dans le groupe comprenant les composés de structures suivantes dans lesquelles Ph signifie phényle :

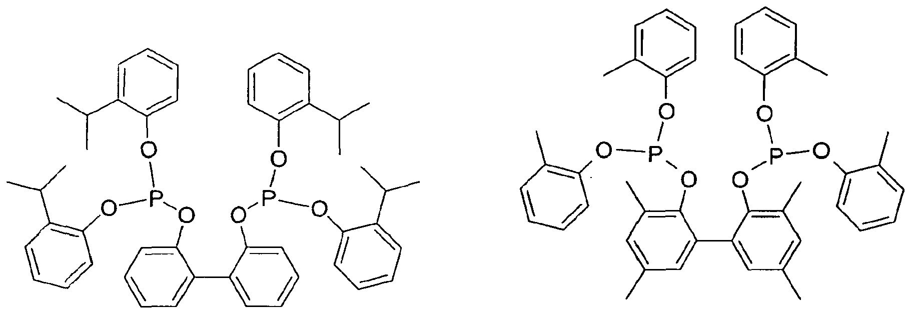

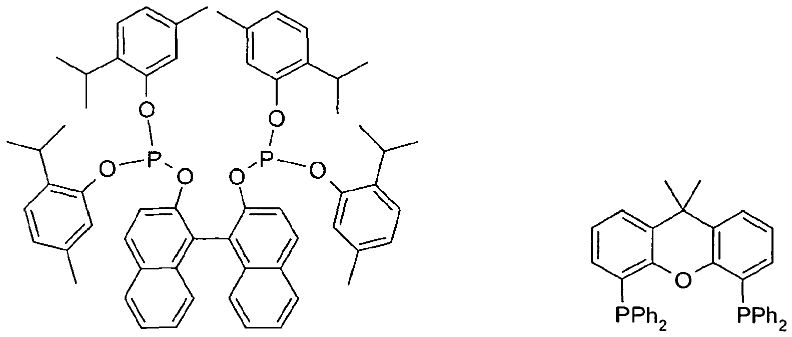

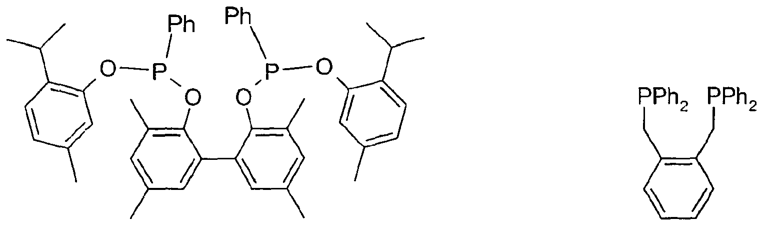

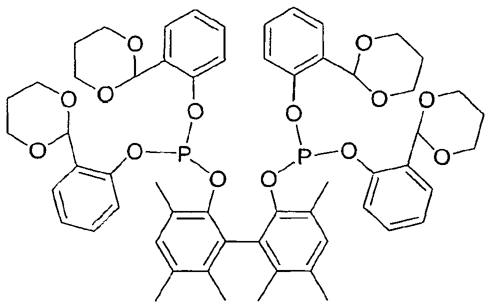

18. Procédé selon l'une des revendications précédentes, **caractérisé en ce que** le système catalytique comprend un promoteur ou cocatalyseur constitué par un acide de Lewis.

19. procédé selon la revendication 18, **caractérisé en ce que** le promoteur est choisi dans le groupe comprenant les composés des éléments des groupes Ib, IIb, IIIa, IIIb, IVa, IVb, Va, Vb, Vlb, Vllb et VIII de la Classification périodique des éléments, lesdits composés étant choisis dans le groupe comprenant les halogénures, sulfates, sulfonates, halogenoalkylsulfonates, perhalogénoalkylsulfonates, halogénoacétates, perhalogénoacétates, carboxylates et phosphates, les aryles boranes.

20. Procédé selon la revendication 19, **caractérisé en ce que** le promoteur ou acide de Lewis est choisi dans le groupe comprenant le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thullium, l'ytterbium et le lutétium, le chlorure de cobalt, le chlorure ferreux, le chlorure d'yttrium, le triphényl borane, le tétraisopropylate de titane et leurs mélanges

21. Procédé selon l'une des revendications 6 à 20, **caractérisé en ce que** la température de pied de colonne dans l'étape de distillation des nitriles insaturés est inférieure à 140°C

22. Procédé selon l'une des revendications précédentes **caractérisé en ce que** le nitrile insaturé est un composé aliphatique linéaire ou ramifié comprenant de 3 à 8 atomes de carbone

23. Procédé selon la revendication 22, **caractérisé en ce que** le nitrile insaturé est un pentènenitrile.

24. Procédé selon l'une des revendications précédentes, **caractérisé en ce que** la solution de système catalytique récupérée après l'étape d'extraction liquide/liquide est soumise à une distillation du solvant d'extraction, la température de pied de cette distillation étant inférieure à 180°C.

25. Procédé de fabrication de composés dinitriles à partir de dioléfines comprenant une première étape d'hydrocyanation de la dioléfine en mononitriles insaturés, éventuellement une réaction d'isomérisation des mononitriles insaturés ramifiés formés à la première étape et une seconde étape d'hydrocyanation des mononitriles insaturés en composés dinitriles selon l'une des revendications 1 à 24.

26. Procédé selon la revendication 25, **caractérisé en ce que** le catalyseur récupéré à partir de la phase supérieure de l'étape de décantation est recyclé au moins partiellement dans la réaction d'isomérisation et/ou à la première étape d'hydrocyanation.

27. Procédé selon la revendication 26, **caractérisé en ce que** le catalyseur récupéré à partir de la phase supérieure de l'étape de décantation est recyclé au moins partiellement dans la réaction d'isomérisation puis dans la première étape d'hydrocyanation.

28. Procédé selon la revendication 27, **caractérisé en ce que** le catalyseur récupéré à partir du milieu d'hydrocyanation de la première étape est alimentée dans l'étape de décantation ou dans l'étape d'extraction liquide/liquide.

## Claims

1. Method for preparing dinitrile compounds by means of hydrocyanation of unsaturated mononitrile compounds in the presence of a catalytic system comprising an organometallic complex formed from a metallic element and an admixture of organophosphorus ligands comprising at least one monodentate organophosphite compound and at least one bidentate organophosphorus compound selected from the group comprising organophosphites, organophosphinites, organophosphonites, organophosphines, and optionally a catalyst promoter, the method comprising a recovery of the catalytic system, **characterised in that** it involves controlling the concentration of unsaturated nitriles in the reaction medium originating from the hydrocyanation reaction in order to obtain a concentration by weight of unsaturated nitriles of less than 20% in the medium, supplying the medium in a decantation step in two phases, an upper phase and a lower phase, separating the two phases, the lower phase being at least partially recycled in a hydrocyanation step, the upper phase being subjected to a liquid/liquid extraction in order to extract the organometallic complex and the organophosphorus compounds present in the phase.

2. Method according to claim 1, **characterised in that** the reaction medium is cooled to a temperature between 25°C and 75°C before being introduced in the decantation step.

3. Method according to either claim 1 or claim 2, **characterised in that** the concentration by weight of nickel in the medium supplied in the decantation step is between 0.2 and 2%.

4. Method according to any one of the preceding claims, **characterised in that** the concentration by weight of unsaturated nitriles in the medium supplied in the decantation step is between 4% and 20% by weight.

5. Method according to any one of the preceding claims, **characterised in that** the concentration of unsaturated nitriles in the reaction medium from the hydrocyanation step is less than 20% by weight.

6. Method according to any one of claims 1 to 5, **characterised in that** the reaction medium from the hydrocyanation step is supplied in a distillation step of the unsaturated nitriles, the lower distillation portion being supplied in the decantation step.

7. Method according to any one of claims 1 to 6, **characterised in that** the lower phase of the decantation step is at least partially recycled in the reaction medium from the hydrocyanation.

8. Method according to claim 7, **characterised in that** a portion of the lower phase is recycled in the reaction medium from the hydrocyanation, before the distillation step of the unsaturated nitriles.

9. Method according to any one of the preceding claims, **characterised in that** the number of moles of bidentate ligand expressed as a number of phosphorus atoms relative to a metallic element atom is between 1 and 4 in the hydrocyanation medium.

10. Method according to any one of the preceding claims, **characterised in that** the number of moles of monodentate ligand expressed as a number of phosphorus atoms relative to a metallic element atom in the hydrocyanation medium is between 4 and 7.

11. Method according to any one of the preceding claims, **characterised in that** the solvent used to implement the liquid/liquid extraction of the catalyst and the organophosphorus compounds contained in the upper phase is selected from the group comprising saturated or unsaturated aliphatic or cycloaliphatic hydrocarbons.

12. Method according to any one of the preceding claims, **characterised in that** the catalyst and the organophosphorus ligands extracted in the liquid/liquid extraction step are recovered by evaporation of the extraction solvent.

13. Method according to claim 11 and claim 12, **characterised in that** the extraction solvent is selected from the group comprising hexane, heptane, octane, cyclohexane, cyclopentane, cycloheptane.

14. Method according to any one of the preceding claims, **characterised in that** the number of moles of organophosphorus ligands expressed as a number of phosphorus atoms in the medium supplied in the liquid/liquid extraction step relative to a metallic element atom is greater than 8.

15. Method according to any one of the preceding claims, **characterised in that** the monodentate ligand is a compound selected from the group comprising triphenylphosphite, tritolylphosphite, tricymenylphosphite.

16. Method according to any one of the preceding claims, **characterised in that** the bidentate organophosphorus ligand is selected from the group comprising organophosphites, organophosphonites, organophosphinites, organophosphines.

17. Method according to claim 16, **characterised in that** the bidentate organophosphorus ligand is selected from the group comprising the compounds having the following structures in which Ph signifies phenyl:

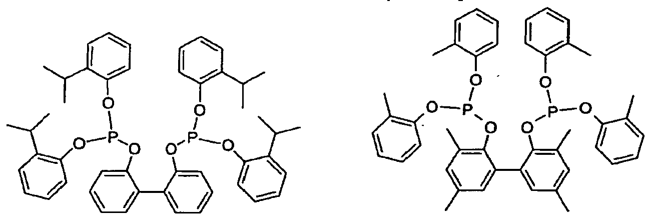

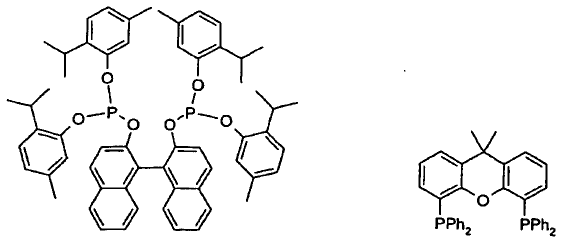

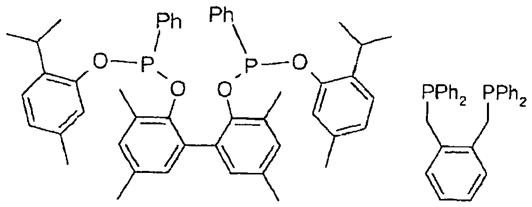

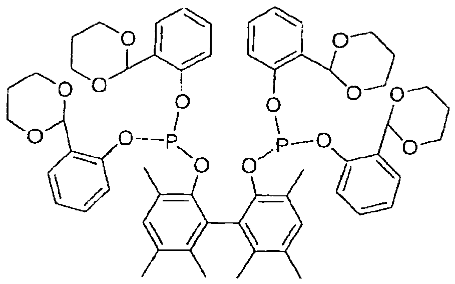

18. Method according to any one of the preceding claims, **characterised in that** the catalytic system comprises a catalyst promoter or cocatalyst which is constituted by a Lewis acid.

19. Method according to claim 18, **characterised in that** the catalyst promoter is selected from the group comprising the compounds of the elements of the groups Ib, IIb, IIIa, IIIb, IVa, IVb, Va, Vb, VIb, VIIb and VIII of the Periodic Classification of elements, the compounds being selected from the group comprising halogenides, sulphates, sulphonates, halogenoalkylsulphonates, perhalogenoalkylsulphonates, halogenoacetates, perhalogenoacetates, carboxylates and phosphates, borane aryls.

20. Method according to claim 19, **characterised in that** the catalyst promoter or Lewis acid is selected from the group comprising zinc chloride, zinc bromide, zinc iodide, manganese chloride, manganese bromide, cadmium chloride, cadmium bromide, stannous chloride, stannous bromide, stannous sulphate, stannous tartrate, indium chloride, indium trifluoromethylsulphonate, indium trifluoroacetate, chlorides or bromides of rare earth elements, such as lanthanum, cerium, praseodymium, neodymium, samarium, europium, gadolinium, terbium, dysprosium, hafnium, erbium, thulium, ytterbium and lutetium, cobalt chloride, ferrous chloride, yttrium chloride, triphenyl borane, titanium tetraisopropylate and the admixtures thereof.

21. Method according to any one of claims 6 to 20, **characterised in that** the temperature of the lower portion of the column in the distillation step of the unsaturated nitriles is less than 140°C.

22. Method according to any one of the preceding claims, **characterised in that** the unsaturated nitrile is a linear or branched aliphatic compound comprising from 3 to 8 atoms of carbon.

23. Method according to claim 22, **characterised in that** the unsaturated nitrile is a pentene nitrile.

24. Method according to any one of the preceding claims, **characterised in that** the catalytic system solution recovered after the liquid/liquid extraction step is subjected to a distillation of the extraction solvent, the temperature of the lower portion of this distillation being less than 180°C.

25. Method for producing dinitrile compounds from diolefins comprising a first step for hydrocyanation of the diolefin into unsaturated mononitriles, optionally an isomerisation reaction of the branched unsaturated mononitriles formed in the first step and a second step for hydrocyanation of the unsaturated mononitriles into dinitrile compounds in accordance with any one of claims 1 to 24.

26. Method according to claim 25, **characterised in that** the catalyst recovered from the upper phase of the decantation step is recycled at least partially in the isomerisation reaction and/or in the first hydrocyanation step.

27. Method according to claim 26, **characterised in that** the catalyst recovered from the upper phase of the decantation step is recycled at least partially in the isomerisation reaction then in the first hydrocyanation step.

28. Method according to claim 27, **characterised in that** the catalyst recovered from the hydrocyanation medium of the first step is supplied in the decantation step or in the liquid/liquid extraction step.

## Patentansprüche

1. Verfahren zur Herstellung von Dinitrilverbindungen durch Hydrocyanierung von ungesättigten Mononitrilverbindungen in Anwesenheit eines katalytischen Systems, das einen metallorganischen Komplex umfasst, der von einem metallischen Element und einem Gemisch von Organophosphat-Liganden gebildet ist, das wenigstens eine Organophosphitmonodentatverbindung und wenigstens eine Organophosphatbidentatverbindung umfasst, die ausgewählt ist aus der Gruppe bestehend aus Organophosphiten, Organophosphiniten, Organophosphoniten, Organophosphinen und eventuell einem Promotor, wobei das genannte Verfahren eine Rückgewinnung des katalytischen Systems beinhaltet, **dadurch gekennzeichnet, dass** es Folgendes beinhaltet: Regeln der Konzentration an ungesättigten Nitrilen im Reaktionsmedium aus der Hydrocyanierungsreaktion, um eine Gewichtskonzentration an ungesättigten Nitrilen von weniger als 20 % in dem Medium zu erzielen, Zuführen des Mediums in einer Dekantierstufe in zwei Phasen, einer hohen und einer tiefen, Trennen der beiden Phasen, wobei die tiefe Phase wenigstens teilweise in einer Hydrocyanierungsstufe recycliert wird, wobei die hohe Phase einer Flüssig/Flüssig-Extraktion unterzogen wird, um den metallorganischen Komplex und die in dieser Phase vorhandenen Organophosphatverbindungen zu extrahieren.

2. Verfahren nach Anspruch 1, **dadurch gekennzeichnet, dass** das Reaktionsmedium vor dem Einleiten in die Dekantierstufe auf eine Temperatur zwischen 25°C und 75°C gekühlt wird.

3. Verfahren nach Anspruch 1 oder 2, **dadurch gekennzeichnet, dass** die Gewichtskonzentration an Nickel in dem in der Dekantierstufe zugeführten Medium zwischen 0,2 % und 2 % liegt.

4. Verfahren nach einem der vorherigen Ansprüche, **dadurch gekennzeichnet, dass** die Gewichtskonzentration an ungesättigten Nitrilen in dem in der Dekantierstufe zugeführten Medium zwischen 4 % und 20 % liegt.

5. Verfahren nach einem der vorherigen Ansprüche, **dadurch gekennzeichnet, dass** die Konzentration an ungesättigten Nitrilen in dem Reaktionsmedium aus der Hydrocyanierungsstufe niedriger als 20 Gew.-% ist.

6. Verfahren nach einem der Ansprüche 1 bis 5, **dadurch gekennzeichnet, dass** das Reaktionsmedium aus der Hydrocyanierungsstufe in einer Destillationsstufe von ungesättigten Nitrilen zugeführt wird, wobei das untere Destillationsende in der Dekantierstufe zugeführt wird.

7. Verfahren nach einem der Ansprüche 1 bis 6, **dadurch gekennzeichnet, dass** die tiefe Phase der Dekantierstufe wenigstens teilweise in dem Reaktionsmedium aus der Hydrocyanierung recycliert wird.

8. Verfahren nach Anspruch 7, **dadurch gekennzeichnet, dass** ein Teil der tiefen Phase in dem Reaktionsmedium aus der Hydrocyanierung vor der Destillationsstufe der ungesättigten Nitrile recycliert wird.

9. Verfahren nach einem der vorherigen Ansprüche, **dadurch gekennzeichnet, dass** die Bidentatligandenmolzahl, ausgedrückt in Anzahl Phosphoratome, in Bezug auf ein Atom eines metallischen Elements zwischen 1 und 4 im Hydrocyanierungsmedium liegt.

10. Verfahren nach einem der vorherigen Ansprüche, **dadurch gekennzeichnet, dass** die Monodentatligandenmolzahl, ausgedrückt in Anzahl Phosphoratome, in Bezug auf ein Atom eines metallischen Elements im Hydrocyanierungsmedium zwischen 4 und 7 liegt.

11. Verfahren nach einem der vorherigen Ansprüche, **dadurch gekennzeichnet, dass** das zum Durchführen der Flüssig/Flüssig-Extraktion des Katalysators und der in der hohen Phase enthaltenen Organophosphatverbindungen verwendete Lösungsmittel ausgewählt ist aus der Gruppe bestehend aus aliphatischen oder cycloaliphatischen Kohlenwasserstoffen, gesättigt oder nicht.

12. Verfahren nach einem der vorherigen Ansprüche, **dadurch gekennzeichnet, dass** der Katalysator und die in der Flüssig/Flüssig-Extraktionsstufe extrahierten Organophosphat-Liganden durch Verdampfung des Extraktionslösungsmittels zurückgewonnen werden.

13. Verfahren nach Anspruch 11 und 12, **dadurch gekennzeichnet, dass** das Extraktionslösungsmittel ausgewählt wird aus der Gruppe bestehend aus Hexan, Heptan, Octan, Cyclohexan, Cyclopentan, Cycloheptan.

14. Verfahren nach einem der vorherigen Ansprüche, **dadurch gekennzeichnet, dass** die Organophosphatligandenmolzahl, ausgedrückt in Anzahl Phosphoratome, in dem in der Flüssig/Flüssig-Extraktionsstufe zugeführten Medium in Bezug auf ein Atom eines metallischen Elements größer als 8 ist.

15. Verfahren nach einem der vorherigen Ansprüche, **dadurch gekennzeichnet, dass** der Monodentat-Ligand eine Verbindung ist, die ausgewählt ist aus der Gruppe bestehend aus Triphenylphosphit, Tritolylphosphit, Tricymenylphosphit.

16. Verfahren nach einem der vorherigen Ansprüche, **dadurch gekennzeichnet, dass** der Organophosphoatbidentat-Ligand ausgewählt ist aus der Gruppe bestehend aus Organophosphiten, Organophosphoniten, Organophosphiniten, Organophosphinen.

17. Verfahren nach Anspruch 16, **dadurch gekennzeichnet, dass** der Organophosphatbidentat-Ligand ausgewählt ist aus der Gruppe bestehend aus den Verbindungen der folgenden Strukturen, in denen Ph Phenyl bedeutet:

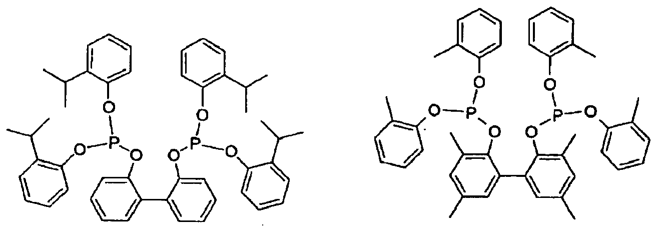

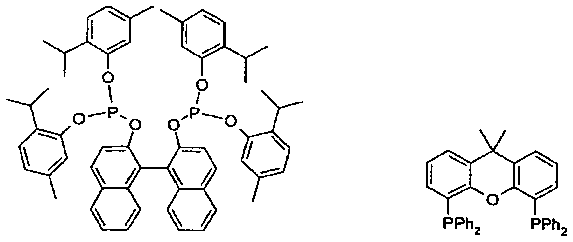

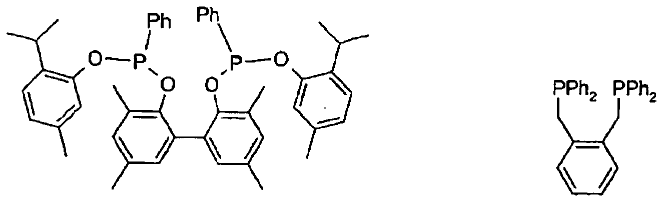

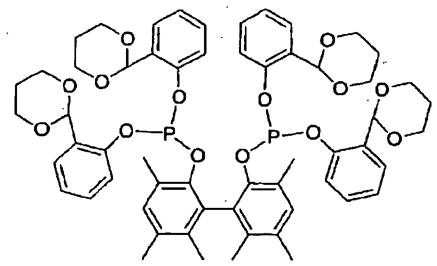

18. Verfahren nach einem der vorherigen Ansprüche, **dadurch gekennzeichnet, dass** das katalytische System einen Promotor oder Co-Katalysator umfasst, der von einer Lewis-Säure gebildet wird.

19. Verfahren nach Anspruch 18, **dadurch gekennzeichnet, dass** der Promotor ausgewählt wird aus der Gruppe bestehend aus den Verbindungen der Elemente der Gruppen Ib, IIb, IIIa, IIIb, IVa, IVb, Va, Vb, VIb, VIIb und VIII des periodischen Elementesystems, wobei die Verbindungen ausgewählt sind aus der Gruppe bestehend aus Halogeniden, Sulfaten, Sulfonaten, Halogenalkylsulfonaten, Perhalogenalkylsulfonaten, Halogenacetaten, Perhalogenacetaten, Carboxylaten und Phosphaten, Boranarylen.

20. Verfahren nach Anspruch 19, **dadurch gekennzeichnet, dass** der Promotor oder die Lewis-Säure ausgewählt ist aus der Gruppe bestehend aus Zinkchlorid, Zinkbromid, Zinkiodid, Manganchlorid, Manganbromid, Cadmiumchlorid, Cadmiumbromid, Zinnchlorid, Zinnbromid, Zinnsulfat, Zinntartrat, Indiumchlorid, Indiumtrifluormethylsulfonat, Indiumtrifluoracetat, Chloriden oder Bromiden von Seltenerdelementen wie Lanthan, Cerium, Praseodym, Neodym, Samarium, Europium, Gadolinium, Terbium, Dysprosium, Hafnium, Erbium, Thullium, Ytterbium und Lutetium, Kobaltchlorid, Eisenchlorid, Yttriumchlorid, Borantriphenyl, Titantetraisopropylat und Gemischen davon.

21. Verfahren nach einem der Ansprüche 6 bis 20, **dadurch gekennzeichnet, dass** die Temperatur um unteren Ende der Säule in der Destillationsstufe von ungesättigten Nitrilen unter 140°C liegt.

22. Verfahren nach einem der vorherigen Ansprüche, **dadurch gekennzeichnet, dass** das ungesättigte Nitril eine lineare oder verzweigte aliphatische Verbindung mit 3 bis 8 Kohlenstoffatomen ist.

23. Verfahren nach Anspruch 22, **dadurch gekennzeichnet, dass** das ungesättigte Nitril ein Pentennitril ist.

24. Verfahren nach einem der vorherigen Ansprüche, **dadurch gekennzeichnet, dass** die nach der Flüssig/Flüssig-Extraktionsstufe zurückgewonnene Katalysesystemlösung einer Destillation des Extraktionslösungsmittels unterzogen wird, wobei die Temperatur am unteren Ende dieser Destillation unter 180°C liegt.

25. Verfahren zur Herstellung von Dinitrilverbindungen von Diolefinen, das eine erste Stufe der Hydrocyanierung des Diolefins in ungesättigten Mononitrilen, eventuell eine Isomerisationsreaktion von in der ersten Stufe gebildeten verzweigten ungesättigten Mononitrilen und eine zweite Hydrocyanierungsstufe von ungesättigten Mononitrilen in Dinitrilverbindungen nach einem der Ansprüche 1 bis 24 beinhaltet.

26. Verfahren nach Anspruch 25, **dadurch gekennzeichnet, dass** der von der hohen Phase der Dekantierstufe zurückgewonnene Katalysator wenigstens teilweise in der Isomerisationsreaktion und/oder in der ersten Hydrocyanierungsstufe recycliert wird.

27. Verfahren nach Anspruch 26, **dadurch gekennzeichnet, dass** der in der hohen Phase der Dekantierstufe zurückgewonnene Katalysator wenigstens teilweise in der Isomerisationsreaktion, dann in der ersten Hydrocyanierungsstufe recycliert wird.

28. Verfahren nach Anspruch 27, **dadurch gekennzeichnet, dass** der aus dem Hydrocyanierungsmedium der ersten Stufe zurückgewonnene Katalysator in der Dekantierstufe oder in der Flüssig/Flüssig-Extraktionsstufe zugeführt wird.
